# EUROPEAN PATENT APPLICATION

(11) **EP 3 505 190 A1**
(43) Date of publication of application: **03.07.2019**
(21) Application number: 17842913.0
(22) Date of filing: 22.08.2017
(51) Int. Cl.: A61K 49/00, C12N 15/90

(54) **METHOD FOR BUILDING MODEL OF ANIMAL SUFFERING FROM NON-HUMAN MAMMAL OBESITY OR RELATED DISEASE AND USE THEREOF**

(30) Priority: 23.08.2016 CN 201610711407
(71) Applicant: Shanghai Institute of Materia Medica, Chinese Academy of Sciences, Shanghai 201203 (CN)
(72) Inventor: DING, Kan, Shanghai 201203 (CN); LI, Yanling, Shanghai 201203 (CN)
(74) Representative: Rössler, Matthias
(86) International application number: PCT/CN2017/098536
(87) International publication number: WO 2018/036491

(57) **Abstract**

A method for preparing a non-human mammalian animal model suffering from obesity or a related disease, comprising the following steps: (a) providing a non-human mammalian cell, and inactivating Glce gene in the cell, thereby obtaining a non-human mammalian cell with an inactivated Glce gene; and (b) using the cell with the inactivated Glce gene obtained in step (a) to prepare and obtain an animal model suffering from Glce gene-inactivated obesity or related diseases. The animal model is an effective animal model suffering from obesity or a related disease, and is useful for researching diseases such as obesity, hyperlipidemia, hypertension, and diabetes, and is useful for in experiments for screening and testing specific medicines.

Sequence listing

<110>  SHANGHAI INSTITUTE OF MATERIA MEDICA, CHINESE ACADEMY OF SCIENCES

<120>  METHOD FOR ESTABLISHING A NON-HUMAN MAMMALIAN ANIMAL MODEL SUFFERING
FROM OBESITY OR RELATED DISEASE AND USE THEREOF

<130>  P2017-1606

<150>  201610711407.4
<151>  2016-08-23

<160>  4

<170>  PatentIn version 3.5

<210>  1
<211>  27
<212>  DNA
<213>  artificial sequence

<220>
<223> primer

<400>  1
ggcatttgca ctcacataca caaccca                                          27


<210>  2
<211>  23
<212>  DNA
<213>  artificial sequence

<220>
<223> primer

<400>  2
gtgccactcc cactgtcctt tcc                                              23


<210>  3
<211>  27
<212>  DNA
<213>  artificial sequence

<220>
<223> primer

<400>  3
gagaggcttg gaggcggtgc tgatctt                                          27


<210>  4
<211>  27
<212>  DNA
<213>  artificial sequence

<220>
<223> primer

<400>  4
gatatactat gccgatgatt aattgtc                                          27

## Description

### Technical field

The present invention relates to the field of biotechnology, and in particular to a method for establishing a non-human mammalian animal model suffering from obesity or related disease and use thereof.

### Background

Obesity is a common, ancient metabolic syndrome. When the body intakes more calories than it consumes, the excess calories are stored in the body in the form of fat, which exceeds the normal physiological requirements, and when it reaches a certain value, it evolves into obesity. The weight of adipose tissue in normal male adult is about 15-18% of body weight, and in women, it is about 20-25%. As age increases, the proportion of body fat increases accordingly. Obesity is defined when body fat increases so that body weight exceeds 20% of standard body weight or body mass index (BMI = body weight (Kg)/(height)²(m²)) is greater than 24. If there is no obvious cause, it is defined as simple obesity, and if there is a clear cause, it is defined as secondary obesity. According to the World Health Organization, obesity is the most unnoticeable disease encountered by human whose incidence is rising sharply.

At present, the pathogenesis of obesity has not been studied clearly, and there is no safe and effective treatment. Therefore, in-depth study of its pathogenesis is an important basis for obesity treatment. The mouse disease model plays a very important role in studying the pathogenesis of human diseases and drug screening. The mouse model has great advantages in exploring human cognitive functions, neurodegenerative diseases, metabolic diseases, digestive diseases and the like.

Gene knockout, also known as "gene targeting" technology, is a complex molecular biology technique developed in the 1980s, which is based on the principle of DNA homologous recombination of mouse embryonic stem cells. Since then, thousands of mutant mice have been constructed by using this technology. These genetically engineered mice not only have brought breakthroughs in biological science and medical research, but also have played a vital role in development of new medicine.

At present, the establishment of mouse obesity model mainly includes food-feeding, hypothalamic and endocrine obesity models and the like, but the production of obesity model is very time-consuming, cumbersome with low success rate, unable to meet requirements for studying obesity mechanisms and developing different types of diet medicine.

Therefore, there is an urgent need in the art to develop an animal model that can better simulate clinical obesity, has small individual differences, and has a highly consistent genetic background, and can be used as a powerful tool for studying the pathogenesis of obesity and screening new drugs.

### Summary of the invention

The object of the present invention is to provide an animal model that can better simulate clinical obesity, has small individual differences, and has a highly consistent genetic background, and can be used as a powerful tool for studying the pathogenesis of obesity and screening new drugs.

In the first aspect of the invention, it provides a method for preparing a non-human mammalian animal model of obesity or related diseases thereof, comprising the steps of:
(a) providing a non-human mammalian cell, and inactivating a *Glce* gene in the cell, thereby obtaining a non-human mammalian cell with an inactivated *Glce* gene;
(b) using the cell with an inactivated *Glce* gene obtained in step (a) to obtain the animal model of obesity or related diseases thereof with the inactivated *Glce* gene.

Preferably, in step (a), it further comprises the following step:
(a1) deleting or interrupting one or more exons in exon 3 to exon 5 of *Glce* gene, and replacing with a selection marker by using DNA homologous recombination techniques, thereby obtaining a non-human mammalian cell with the inactivated *Glce* gene.

Preferably, in step (b), it further comprises the following steps:
(b1) preparing and obtaining a chimeric non-human mammal by using the non-human mammalian cell with the inactivated *Glce* gene obtained in step (a);
(b2) mating the chimeric non-human mammal obtained in step (b1) with a normal wild-type non-human mammal, and screening progeny to obtain a heterozygous non-human mammal with the inactivated *Glce* gene;
(b3) mating the heterozygous non-human mammals obtained in step (b2) with each other to obtain a homozygous non-human mammal with an inactivated *Glce* gene, thereby obtaining the non-human mammalian animal model with the inactivated *Glce* gene.

Preferably, in step (b3), it further comprises a step of (b4): hybridizing the homozygous non-human mammal with an inactivated *Glce* gene with a neuron-specific knockout tool model which is a non-human mammal of the same species, thereby obtaining a non-human mammalian animal model of with a neuron-specifically inactivated *Glce* gene.

Preferably, the inactivation of *Glce* gene comprises gene knockout, gene disruption or gene insertion.

Preferably, the inactivation of gene comprises non-expression of *Glce* gene or expression of an inactive Glce protein.

Preferably, the inactivation of Glce gene is achieved by deletion or knockout of exon 3 of *Glce.*

Preferably, the inactivation of Glce gene is a neuron-specifically inactivation of *Glce* gene.

Preferably, the non-human mammal is rodent or primate, and more preferably comprises mouse, rat, rabbit, and/or monkey.

Preferably, the non-human mammal is mouse, and in step (b4), the Glce Loxp/Loxp mouse is mated with a tool mouse NSE (neuron-specific enolase)-Cre, thereby obtaining a neuron-specific Glce knockout mouse, referred as cKO mouse (i.e., mouse with neuron-specifically inactivated Glce).

Preferably, the selection marker is selected from the group consisting of: a resistance gene, a fluorescent protein gene, and combinations thereof.

Preferably, the selection marker comprises a *neo* gene.

Preferably, compared with the wide type control animal, the non-human mammalian animal model with the inactivated Glce gene obtained in step (b) has one or more characteristics selected from the group consisting of:
(t1) an increase in degree of obesity;
(t2) an increase in incidence of obesity symptom;
(t3) an increase in wet weight of adipose tissue;
(t4) a decrease in level of open field activity;
(t5) a reduced desire to explore new environment;
(t6) an increase in depression-like behavior;
(t7) an increase in degree of depression;
(t8) an increase in anxiety-like behavior;
(t9) an increase in degree of anxiety;
(t10) an increased in fear-like behavior;
(t11) an increase in degree of fear;
(t12) an increase in cognitive disorder;
(t13) an increase in incidence of stroke.

Preferably, the wet weight of adipose tissue is selected from the group consisting of: wet weight of visceral adipose tissue, wet weight of subcutaneous fat tissue, and combinations thereof.

Preferably, the wet weight of the visceral adipose tissue is selected from the group consisting of: wet weight of bilateral perigonadal adipose tissue, wet weight of bilateral perirenal adipose tissue, and combinations thereof.

Preferably, the wet weight of the subcutaneous adipose tissue comprises the wet weight of bilateral inguinal adipose tissue.

Preferably, the open field activity level is selected from the group consisting of: a distance, an activity time, an activity speed of the open field activity, and combinations thereof.

Preferably, the depression-like behavior is selected from the group consisting of: a decrease of time to explore the central region in the open field experiment, a decrease of desire to explore a new environment, and an increase of immobility time in a forced swimming experiment, appearance of behavioral despair, and combinations thereof.

In the second aspect of the invention, it provides a use of a non-human mammalian model prepared by the method of the first aspect of the invention for studying obesity or related diseases thereof wherein it is used as an animal model.

Preferably, the obesity or related diseases thereof is selected from the group consisting of obesity, hyperlipidemia, hypertension, diabetes, and combinations thereof.

In the third aspect of the invention, it provides a use of a non-human mammalian model prepared by the method of the first aspect of the invention for screening or identifying a substance (or a therapeutic agent) that relieves or treats obesity or related diseases thereof.

Preferably, the obesity or related diseases thereof is selected from the group consisting of obesity, hyperlipidemia, hypertension, diabetes, and combinations thereof.

In the fourth aspect of the invention, it provides a method of screening or identifying a potential therapeutic agent for treating or relieving obesity or related diseases thereof, comprising the steps of:
(a) in a test group, in the presence of a test compound, the test compound is administered to a non-human mammalian animal model prepared by the method of the first aspect of the invention, and the severity Q1 of obesity or related diseases thereof in animal model of the test group is measured; and in the control group in which the test compound is not administered and the other conditions are the same, the severity Q2 of obesity or related diseases thereof in the animal model of the control group is measured;
(b) comparing the severity Q1 and severity Q2 measured in the previous step to determine whether the test compound is a potential therapeutic agent for treating or relieving obesity or related diseases thereof;
wherein, if the severity Q1 is significantly lower than the severity Q2, it indicates that the test compound is a potential therapeutic agent for treating or relieving obesity or related diseases thereof.

Preferably, the detection of the severity of obesity or related diseases thereof comprises the detection of a change in one or more indicators selected from the group consisting of: a morphological change, a change in the incidence of obesity symptom, and a change in wet weight of adipose tissue.

Preferably, the morphological change comprises an increase in degree of morphological obesity.

Preferably, the wet weight of adipose tissue is selected from the group consisting of: wet weight of visceral adipose tissue, wet weight of subcutaneous fat tissue, and combinations thereof.

Preferably, the wet weight of the visceral adipose tissue is selected from the group consisting of: wet weight of bilateral perigonadal adipose tissue, wet weight of bilateral perirenal adipose tissue, and combinations thereof.

Preferably, the wet weight of subcutaneous adipose tissue comprises wet weight of the bilateral inguinal adipose tissue.

Preferably, the reduction in the severity of obesity or related diseases thereof is manifested by a reduction in the degree of morphological obesity, a decrease in incidence of obesity symptom, and/or a decrease in wet weight of adipose tissue.

Preferably, the "significantly lower" means that the severity Q1 in the test group having biological repeatability is lower than the severity Q2 in the control group having biological repeatability after administrating the test compound and the P value is less than 0.05 in a *t*-test.

Preferably, the "significantly lower" means that the ratio of severity Q1/severity Q2 is ≤ 1/2, preferably ≤ 1/3, and more preferably ≤ 1/4.

Preferably, the method is non-diagnostic and non-therapeutic.

Preferably, the method comprises a step of (c): applying the potential therapeutic agent screened or identified in step (b) to a non-human mammalian model prepared by the method of the first aspect of the invention, thereby determining its effects on the severity of obesity or its related diseases in the animal model.

In the fifth aspect of the invention, it provides a use of a cell in which a Glucuronyl C5-epimerase (*Glce*) gene is inactivated or down-regulated for preparing a biological formulation for construction of a non-human mammalian animal model of obesity or related diseases.

Preferably, the biological formulation is a liquid formulation.

In the sixth aspect of the invention, it provides a use of an inactivating agent of *Glce* gene or a protein thereof for preparation of a formulation for constructing a non-human mammalian animal model of obesity or related diseases.

Preferably, the inactivating agent comprises an inhibitor.

Preferably, the inactivating agent is selected from the group consisting of: an antibody, a small molecule compound, a nucleic acid, and combinations thereof.

In the seventh aspect of the invention, it provides a non-human mammalian model prepared by the method of the first aspect of the invention.

Preferably, the non-human mammalian model is heterozygous or homozygous for the *Glce* gene inactivation.

Preferably, the *Glce* gene inactivation is a neuron-specific inactivation of *Glce* gene.

It should be understood that, within the scope of the present invention, each technical feature of the present invention described above and in the following (as examples) may be combined with each other to form a new or preferred technical solution, which is not listed here due to space limitations.

### Description of Figure

Figure 1 shows design strategy for Glce gene mutation sequence.
Figure 2 shows a plasmid map of the Glce knockout targeting vector.
Figure 3 shows enzyme digestion results of plasmid DNA of the Glce gene knockout targeting vector.
Figure 4 shows electrophoresis results of the 5'-end PCR product of ES cloned genomic DNA.
Figure 5 shows electrophoresis results of the 3'-end PCR product of ES cloned genomic DNA.
Figure 6 shows PCR results of the Glce transgenic mouse (Loxp/+) sequence mutation site.
Figure 7 shows PCR results of sequence mutation sites of Glce mutant homozygotes (Loxp/Loxp Cre), mutant heterozygotes (Loxp/+ Cre) and Wild type (+/+ Cre).
Figure 8 shows a morphological comparison of Glce mutant homozygous mice and C57 mice. The left panel: the upper is C57 female mice, the lower is Glce mutant homozygous female mice. The right panel: the upper is C57 male mice, the lower is Glce mutant homozygous male mice.
Figure 9 shows a comparison of body weight between Glce mutant homozygous mice and C57 mice.
Figure 10 shows a comparison of the body fat wet weight of Glce mutant homozygous mice and C57 mice.
Figure 11 shows a comparison of the wet weight of the bilateral perigonadal adipose tissue in Glce mutant homozygous mice and C57 mice.
Figure 12 shows a comparison of wet weight of bilateral perirenal adipose tissue in Glce mutant homozygous mice and C57 mice.
Figure 13 shows a comparison of the wet weight of visceral adipose tissue in Glce mutant homozygous mice and C57 mice.
Figure 14 shows a comparison of wet weight of bilateral inguinal adipose tissue in Glce mutant homozygous mice and C57 mice.
Figure 15 shows the spontaneous activity distance, time and speed of Glce mutant homozygous mice.
Figure 16 shows the total distance, time and velocity of Glce mutant homozygous mice in open field experiments.
Figure 17 shows the activity distance, time and velocity of Glce mutant homozygous mice in the central region of the open field.
Figure 18 shows the activity distance, time and velocity of Glce mutant homozygous mice in the surrounding area of the open field.
Figure 19 shows the immobility time of Glce mutant homozygous mice in a forced swimming experiment.
Figure 20 shows an experiment for evaluating stroke behavior.
Figure 21 shows the detection of brain tissue infarction in stroke mice by TTC staining.
Figure 22 shows the spontaneous activity distance, time and speed of Glce mutant homozygous mice.
Figure 23 shows the activity distance, time and velocity of Glce mutant homozygous mice in the central region of the open field.
Figure 24 shows the time of the open arms, central region, and closed arms of the Glce mutant homozygous mice in an elevated cross maze experiment.

### Detailed Description

After an extensive and intensive study, the present inventors have established a genetically stable, phenotypically stable obesity model, which is a mouse or other non-human mammal animal whose Glce gene has been knocked out or inactivated. The animal model of the present invention is an effective animal model of obesity or related diseases thereof, and can be used for studying obesity-related diseases such as hyperlipidemia, and is useful for screening and testing of specific medicines. On this basis, the inventors have completed the present invention.

In addition, the present inventors have unexpectedly discovered that an animal model obtained by knocking out or inactivating Glce gene can also be used for studying diseases such as stroke, depression, anxiety and the like. On this basis, the inventors have completed the present invention.

### Glce gene

Heparan sulfate is a polysaccharide widely present on cell surface and in cytoplasmic matrix. As a negatively charged linear macromolecule, many cytokines, growth factors, chemokines and interleukins can specifically bind to it, and furthermore playing a role in physiological processes such as embryonic development, cell growth, inflammatory response, coagulation, tumor metastasis and viral infection¹.

Glucuronic acid C5 isomerase (Glce) is a key enzyme in the synthesis of heparan sulfate proteoglycan sugar chain. It is capable of isomerizing D-glucuronic acid in a sugar chain into L-iduronic acid², thereby greatly increasing the complexity of heparan sulfate and improving flexibility of sugar chain. L-iduronic acid is an indispensable site for identification of numerous protein molecules by heparan sulfate.

In a study of 21 normal breast tissues and 74 breast tumor tissues, it was found that the expression of Glce at mRNA and protein levels in 82%-84% of human breast tumor tissues was significantly down-regulated or completely lost⁴. Additionally, overexpression of Glce in breast and lung cancer cell lines can inhibit proliferation of small cell lung cancer and breast cancer cells, suggesting that Glce may be a potential tumor suppressor gene^{5,6}.

The Glce gene is located on chromosome 9 of mouse genome with a full length of 618 (EnsemblGene ID: ENSMUSG00000032252, Genebank accession number: 93683). The Glce genome sequence comprises 4 introns and 5 exons. There are 3 transcripts of Glce gene for expressing protein, wherein transcript 1 has 3 exons, and 2 introns; transcript 2 has 5 exons, and 4 introns; and transcript 3 has two exons and one intron. These sequence information can be found in the literature or in public databases such as EnsemblGene and Genebank.

The Glce gene of other species such as human can also be found in the literature or in public databases such as EnsemblGene and Genebank.

It should be understood that the term "Glce" also includes variant forms of the various naturally occurring Glce genes. Representative examples include a nucleotide sequence encoding a Glce protein identical to the wild type due to degeneracy of the codon, and a nucleotide sequence encoding a conservative variant polypeptide of the wild type Glce protein. Furthermore, for any mammals other than mice, the term refers to homologs of the Glce gene in that mammal. For example, for human, the term refers to human Glce (cDNA homology between the known mouse Glce gene and human Glce gene is 91.4%, and the homology of amino acid sequence is 97.4%).

### Inactivating agent of Glce gene or protein thereof

In the present invention, the inactivation of the Glce includes all inactivation or partial inactivation.

The inactivating agent of Glce protein in the present invention includes (a) an inhibitor, which, e.g., includes, but is not limited to, a small molecule compound, an antibody, an antisense nucleic acid, a miRNA, a siRNA, and combinations thereof; (b) Glce gene knockout agent.

### Obesity or related diseases

When the body intakes more calories than it consumes, the excess calories are stored in the body in the form of fat, which exceeds the normal physiological requirements, and when it reaches a certain value, it evolves into obesity. The weight of adipose tissue in normal male adult is about 15-18% of body weight, and in women, it is about 20-25%. As age increases, the proportion of body fat increases accordingly. Obesity is defined when body fat increases so that body weight exceeds 20% of standard body weight or body mass index (BMI = body weight (Kg)/(height)² (m²)) is greater than 24. If there is no obvious cause, it defined as simple obesity, and if there is a clear cause, it is defined as secondary obesity. According to the WHO, obesity is the most unnoticeable disease encountered by human whose incidence is rising sharply..

According to statistics, in obese subject, the incidence of cerebral embolism and heart failure is 1 fold higher than that in a subject with normal weight, incidence of coronary heart disease is 2 folds higher than that in a subject with normal weight, incidence of hypertension is 2-6 folds higher than that in a subject with normal weight, incidence of diabetes is about 4 folds higher than that in a subject with normal weight, and incidence of cholelithiasis is 4-6 folds higher than that in a subject with normal weight. More severely, the lifespan of obese people is significantly shortened. It is reported that a 45-year-old male with a 10% overweight has a lifespan which is 4 years shorter than that that in a subject with normal weight. According to Japanese statistics, if the standard mortality rate is 100%, the obese mortality rate is 127.9%.

In the present invention, obesity or related diseases include, but are not limited to, obesity, hyperlipemia, hypertension, and/or diabetes.

### Gene inactivation

For the study of functionally unknown genes, various methods are useful, such as inactivating genes to be studied, analyzing the resultant phenotypic changes of genetic modification, and obtaining functional information of genes. Another advantage of such research method is that it can correlate gene function with disease, so that when the gene function is obtained, the disease information and disease animal model, in which the gene can be treated as a potential drug or drug target, is also obtained. Gene inactivation can be accomplished by gene knockout, gene disruption, or gene insertion, wherein gene knockout technology is a very powerful means to study the function of human genes in a whole body.

### Animal model

In the present invention, a very effective non-human mammalian model of obesity or related diseases thereof is provided.

In the present invention, examples of non-human mammals include, but are not limited to, mice, rats, rabbits, monkeys, and the like, and more preferably rats and mice.

As used herein, the term "Glce gene inactivation" includes the case where one or both Glce genes are inactivated, i.e., including the heterozygous and homozygous inactivation of the Glce gene. For example, a mouse with an inactivated Glce gene can be a heterozygous or homozygous mouse.

In the present invention, a non-human mammal (e.g., a mouse) in which the Glce gene is inactivated can be produced by gene knockout or by transferring and inserting a foreign gene (or fragment) to inactivate Glce gene. Techniques for inactivating target genes by gene knockout or by transferring and inserting foreign genes are known in the art, and these conventional techniques can be used in the present invention.

In another preferred embodiment of the invention, inactivation of the Glce gene is achieved by gene knockout.

In another preferred embodiment of the invention, inactivation of Glce gene is achieved by insertion of a foreign gene (or fragment) into the Glce gene.

In a specific embodiment of the invention, a construct comprising an exogenous insert fragment is constructed, which comprises homology arms homologous to two flanking sequences flanking the insertion site of a target gene (Glce), thereby allowing high frequency insertion of exogenous inserts (or genes) into the Glce genome sequence (especially exon regions) by homologous recombination, resulting in frame shift, early termination, or knockout of the mouse Glce gene, so as to cause a loss or inactivation of Glce.

The homozygous or heterozygous mouse obtained by the method of the present invention is fertile and develops normally. The inactivated Glce gene can be inherited to the offspring mice by Mendelian law.

In a preferred embodiment, the present invention provides a homozygous mouse model animal with Glce gene deletion.

### Candidate medicine or therapeutic agent

In the present invention, it also provides a method of screening for a candidate medicine or a therapeutic agent for treating obesity or related diseases thereof using the animal model of the present invention.

In the present invention, a candidate medicine or therapeutic agent refers to a substance which is known to have a certain pharmacological activity or is being tested and which may have a certain pharmacological activity. The examples include but are not limited to nucleic acid, protein, chemically synthesized small molecule or large molecular compound, cell, and the like. The candidate medicine or therapeutic agent can be administered orally, intravenously, intraperitoneally, subcutaneously, spinally, or direct intracerebral injection.

The main advantages of the invention include:
(1) The present invention can better simulate primary obesity in clinic.
(2) There is no difference in the surgical procedure or drug dose among subjects, and the genetic background is highly consistent.
(3) It is useful as a powerful tool to study pathogenesis of obesity and screen new drugs.
(4) The obesity model of the invention is genetically stable and phenotypically stable.
(5) A homozygous or heterozygous animal model obtained by the method of the invention is fertile and develops normally. Transgenic heterozygous male mice have reproductive ability, and the inactivated Glce gene can be inherited to the offspring mice by Mendelian law.
(6) The animal model of obesity or related diseases of the present invention exhibits symptoms such as obesity, hyperlipemia, hypertension, and the like, and thus can be widely used for screening and testing a medicine for obesity-related diseases.
(7) The present invention discloses for the first time that an animal model obtained by knocking out or inactivating Glce gene can also be used for studying certain diseases such as stroke, depression, and anxiety.

The present invention is further described below with reference to specific embodiments. It should be understood that these examples are only for illustrating the present invention and not intended to limit the scope of the present invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions proposed by the manufacturer. Unless otherwise indicated, percentages and parts are by weight and parts by weight.

Unless otherwise specified, materials and reagents used in the examples are all commercially available products.

### Example 1 Obtaining a homozygous mouse having Glee gene mutation and carrying Cre recombinase

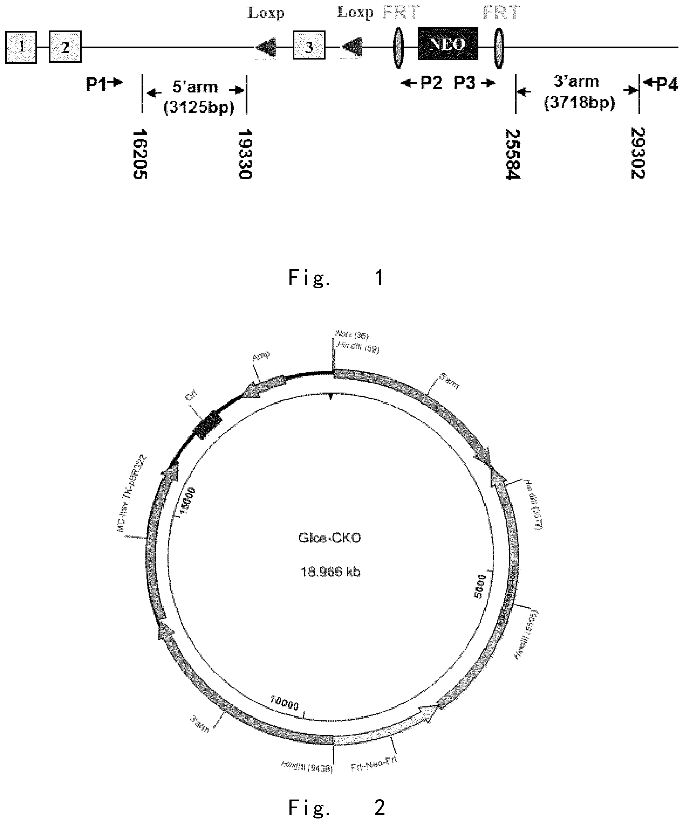

The Glce mutant gene sequence was first constructed (Fig. 1). The Glce knockout targeting vector sequence was designed as shown in Figure 1. The Loxp/Loxp alleles were inserted at both sides of exon 3 of Glce gene, and *neo* gene was inserted at the 3' end. The 5' end arm was 3125 bp and the 3' end arm was 3718. Figure 2 shows a plasmid map of the Glce knockout targeting vector. 1. A homologous fragment of the gene of interest (Glce) was obtained. The DNA fragment was cloned into a plasmid vector. 2. Most of the homologous DNA sequence of the gene of interest was cut off from the recombinant plasmid, leaving only part of the sequence on both sides of linear plasmid vector. 3. The *neo* gene was cloned into a linear plasmid carrying the homologous sequence of the gene of interest so that it was located in the middle of the residual homologous sequence of gene; 4. The recombinant plasmid vector was linearized by cutting the outside the homologous sequence of the target gene and the hsv-tk gene was cloned into this linear vector.

The gene sites are shown in the following table:

| Glce-CKO-vector | | | |
|---|---|---|---|
| Start | End | Name/description | Gene site |
| 1 | 57 | Cloning site (57) | |
| 64 | 3189 | 5'arm (3125) | 16205-19330 |
| 3222 | 7577 | Loxp-Exon3-Loxp (4355) | |
| 7596 | 9423 | Frt-Neo-Frt (1827) | |
| 9443 | 13161 | 3'arm (3718) | 25584-29302 |
| 13168 | 18966 | MC-hsv TK-pBR322 (5798) | |

| | | | |
|---|---|---|---|
| Note: The gene position numbering is based on "10kb Up and Down of Glce gene". | | | |

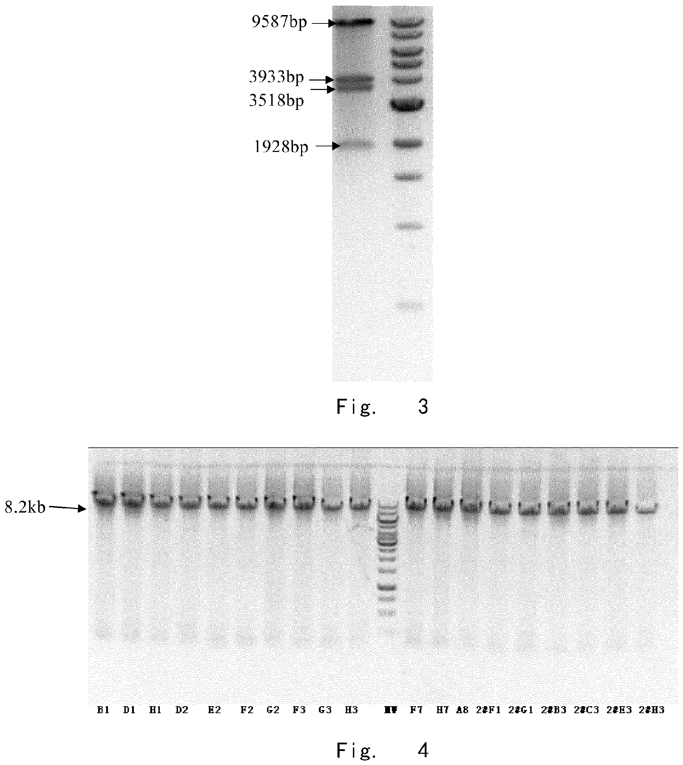

Figure 3 shows the enzyme digestion of Glce gene knockout targeting vector plasmid DNA using a 1 Kb DNA ladder. The targeting vector was linearized: 100 µg of Glce-CKO plasmid DNA (purchased from Biovector NTCC) was linearized by using NotI (enzyme dosage: 150 U) in a 150 µl enzyme digestion system, and digested overnight at 37 °C. After treatment with an equal volume of phenol chloroform and chloroform, the DNA was precipitated with absolute ethanol and resuspended in 100 µl sterile PBS (ready for use). ES cell targeting operation: ES cell SCR012 was derived from embryonic stem cells of 129SV/EV strain male mice (purchased from Shanghai Experimental Animal Center of Chinese Academy of Sciences). Linearized DNA amount: 35µg; electro-transferring device model: Bio-Rad Gene Pulser (Cat. No. 165-2105), electroporation conditions: voltage: 240V, capacitance: 500 µF, actual energization time: 10.5 ms, actual voltage: 256V. Clonal screening conditions were 300 µg/ml G418 and 2 µM GanC for 8 days. A total of 96 copies of resistant clones and DNA samples were obtained.

Positive ES cell genome identification method:

### 1. PCR identification for 5'arm

The P1 primer was located outside the 5'arm, and the P2 primer was located in the neo recombination region, and was 8.2 kb away from the extra-arm primer.

P1 and P2 primer sequences:
P1: GGCATTTGCACTCACATACACAACCCA (gene site:15824-15850) (SEQ ID NO.:1)
P2: GTGCCACTCCCACTGTCCTTTCC (SEQ ID NO.:2)

**PCR reaction system(ul):**

| | |
|---|---|
| La Taq | 0.3 |
| 10X La Buffer | 2.5 |
| dNTP (2.5µM) | 2.5 |
| ES genomic DNA | 1.0 |
| P1 primer(10µM) | 0.5 |
| P2 primer(10µM) | 0.5 |
| ddH₂O | 17.7 |
| Total | 25.0 |

**PCR reaction conditions:**

| | | | |
|---|---|---|---|
| 95°C | 3min | | |
| 95°C | 30s | 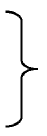 | 35 cycles |
| 63°C | 30s | | |
| 72°C | 5min | | |
| 72°C | 10min | | |
| 12°C | | | |

PCR instrument: Eppendorf AG 22331 from Hamburg
Reagent: TaKaRa La Taq was from Bao Bioengineering (Dalian) Co., Ltd. (Cat: DRR002B)
Molecular weight Marker: MBI GeneRuler 1kb DNA Ladder (Jingmei biology, Cat: SM0311)

### 2. PCR identification for 3'arm

The P4 primer was located outside the 3' arm, and the P3 primer was located in neo recombination region and was 4.7 kb away from the extra-arm primer.

P4 and P3 primer sequences:
P4: GAGAGGCTTGGAGGCGGTGCTGATCTT (gene site:29603-29629) (SEQ ID NO.:3)
P3: GATATACTATGCCGATGATTAATTGTC(SEQ ID NO.:4)

**PCR reaction system (ul):**

| | |
|---|---|
| La Taq | 0.3 |
| 1 0XLaTaq Buffer | 2.5 |
| dNTP (2.5 µM) | 2.5 |
| ES genomic DNA | 1.0 |
| P3 primer(10 µM) | 0.5 |
| P4 primer(10 µM) | 0.5 |
| ddH₂O | 17.7 |
| total | 25.0 |

**PCR reaction conditions:**

| | | | |
|---|---|---|---|
| 95°C | 2min | | |
| 95°C | 45s | 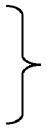 | 35 cycles |
| 65°C | 5min | | |
| 72°C | 10min | | |
| 12°C | | | |

PCR instrument: Eppendorf AG 22331 from Hamburg
Reagent: TaKaRa La Taq from Bao Bioengineering (Dalian) Co., Ltd. (Cat: DRR002B)
Molecular weight Marker: MBI GeneRuler 1kb DNA Ladder (Jingmei biology, Cat: SM0311)

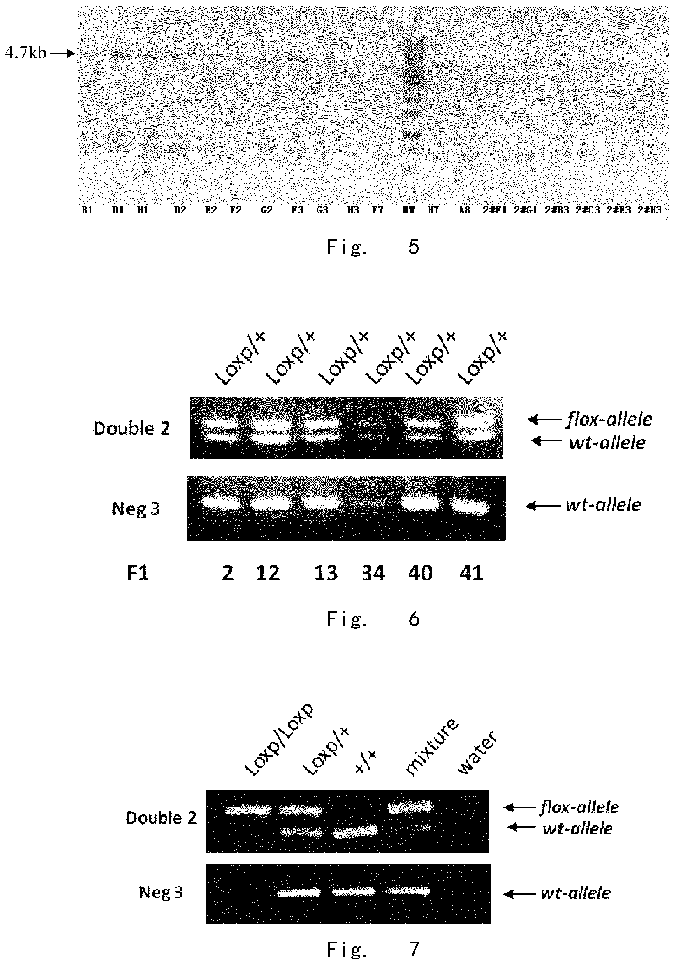

ES cell cloning identification results: 96 drug-resistant ES cell clones were identified by PCR, wherein in 19 ES clones, double-arm homologous recombination occurred. The PCR product was further confirmed by DNA sequencing. Figure 4 showed the electrophoresis results of the 5'-end PCR product of ES cloned genomic DNA. Figure 5 showed the electrophoresis results of the 3'-end PCR product of ES cloned genomic DNA.

### Positive ES clonal blastocyst injection:

Source of blastocyst for microinjection: C57BL/6J mice (purchased from Shanghai Slack Laboratory Animal Co., Ltd.) were superovulated and naturally fertilized to the blastocyst stage. 60 embryos were injected and transplanted into the recipient uterus of three pseudopregnant mice. The recipients were the 1^{st} generation of hybrids of C57BL/6J (♂) and CBA (♀) (purchased from Shanghai Slack Laboratory Animal Co., Ltd.). Mice with a chimeric rate higher than 50% were selected from the born mice and raised to adulthood. They were mated with C57BL/6J female mice, and the gray mice of the offspring were identified by PCR using the extracted genomic DNA from tail (identification strategy was the same as above). As shown in Figure 6, two-arm positive F1 mice (Loxp/+) were obtained.

F1 generation mice were raised to adulthood, and hybridized with NSE-Cre tool mice (purchased from Shanghai Slack Laboratory Animal Co., Ltd.) to obtain F2 generation Loxp/+ Cre mice. The F2 generation mice were raised to adulthood, and the males and females of the F2 generation were mated each other. According to the Mendelian heredity law, the ratio of F3 generation mutant homozygotes (Loxp/Loxp Cre): mutation heterozygotes (Loxp/+ Cre): Wild type (+/ + Cre) was approximately 1:2:1. The tail genomic DNA was extracted for PCR identification (the identification strategy was the same as above), and the results were shown in FIG. 7. Mutant homozygous mice (Loxp/Loxp Cre) were used for subsequent experiments on animal behaviors.

### Example 2 Morphological analysis of Glee mutant homozygous mice

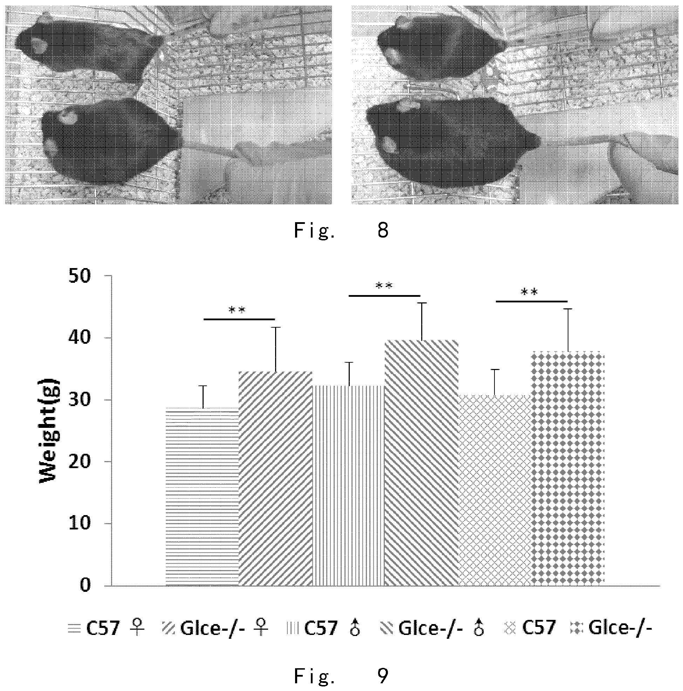

The birth rate of Glce mutant homozygous mice was in accordance with Mendel's law, and the results were shown in Fig. 8. The results showed that the morphology of adult Glce neural-specific knockout mice was more obese than that of adult C57BL/6 normal mice.

### Example 3 Body weight analysis of Glee mutant homozygous mice

Glce mutant homozygous mice were weighed on an electronic balance to study the difference in body weight between adult Glce mutant homozygous mice and adult C57BL/6 normal mice.

The study showed (Fig. 9) that the adult Glce mutant homozygous mice had a higher incidence of obesity symptom as compared with adult C57BL/6 normal mice, and the body weight was statistically significantly different from that of adult C57BL/6 normal mice.

The results showed that when obesity was defined as more than 20% of the average body weight of adult C57BL/6 normal mice, the average weight of female adult C57BL/6 normal mice was 28.7 g, the average weight of female adult Glce mutant homozygous mice was 34.6 g, and the incidence of obesity in female adult Glce mutant homozygous mice was about 50%, which was statistically significant as compared with female adult C57BL/6 normal mice (P<0.01); the average weight of male adult C57BL/6 normal mice was 32.2g, the average weight of male adult Glce mutant homozygous mice was 39.5 g, and the incidence of obesity in male adult Glce mutant homozygous mice was about 66%, which was statistically significant as compared with male adult C57BL/6 normal mice (P<0.01); the adult C57BL/6 normal mice (including males and females) had an average body weight of 30.7 g, and the adult Glce mutant homozygous mice (including males and females) had an average body weight of 37.8 g and the incidence of obesity symptom in adult Glce mutant homozygous mice was about 60%, which was statistically significant (P<0.01) as compared with adult C57BL/6 normal mice.

The above results fully indicated that the incidence of obesity symptom in adult Glce mutant homozygous mice was higher than that in adult C57BL/6 normal mice.

### Example 4 Analysis of body fat of Glce mutant homozygous mice

Glce mutant homozygous mice were dissected and adipose tissues including bilateral perigonadal adipose tissue, bilateral perirenal adipose tissue and bilateral inguinal adipose tissue were weighed on an electronic balance and the difference in wet weight of adipose tissue between adult Glce mutant homozygous mice and adult C57BL/6 normal mice was studied.

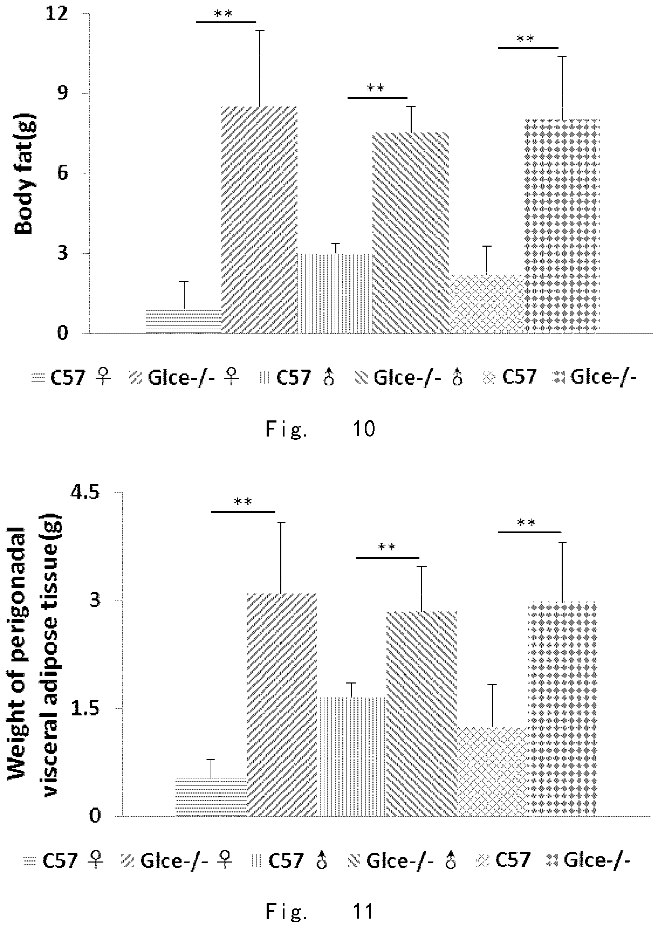

The study showed (Fig. 10) that the adult Glce mutant homozygous mice had a significant increase in wet weight of body fat as compared with adult C57BL/6 normal mice, which was statistically significant.

The results showed that the average body fat wet weight of female adult C57BL/6 normal mice was 0.94 g, and the average body fat wet weight of female adult Glce mutant homozygous mice was 8.50 g, so there was a statistically significant difference as compared with female adult C57BL/6 normal mice (P<0.01); the average body fat wet weight of male adult C57BL/6 normal mice was 2.98 g, and the average body fat wet weight of male adult Glce mutant homozygous mice was 7.52 g, so there was a statistically significant difference as compared with male adult C57BL/6 normal mice (P<0.01); adult C57BL/6 normal mice (including male and female) had an average body fat wet weight of 2.21 g, adult Glce mutant homozygous mice (including male and female) had an average body fat wet weight of 8.01 g, so there was a statistically significant difference (P<0.01) as compared with adult C57BL/6 normal mice.

The above results fully indicated that the body fat wet weight of adult Glce mutant homozygous mice was increased as compared with adult C57BL/6 normal mice.

### Example 5 Adipose tissue analysis of Glce mutant homozygous mice

### 5.1 Analysis of bilateral perigonadal adipose tissue of Glce mutant homozygous mice

The Glce mutant homozygous mice were dissected and the bilateral perigonadal adipose tissue was weighed on an electronic balance and the difference of wet weight of bilateral perigonadal adipose tissue in adult Glce mutant homozygous mice and adult C57BL/6 normal mice was studied..

Compared with adult C57BL/6 normal mice, adult Glce mutant homozygous mice showed a significant increase in wet weight of bilateral perigonadal adipose tissue (Fig. 11).

The results showed that the average wet weight of bilateral perigonadal adipose tissue in female adult C57BL/6 normal mice was 0.54 g, and the average wet weight of the bilateral perigonadal adipose tissue in female adult Glce mutant homozygous mice was 3.10 g, so there was a statistically significant difference as compared with female adult C57BL/6 normal mice (P<0.01); the average wet weight of bilateral perigonadal adipose tissue in male adult C57BL/6 normal mice was 1.66 g, the average wet weight of bilateral perigonadal adipose tissue in male adult Glce mutant homozygous mice was 2.85 g, so there was a statistically significant difference as compared with male adult C57BL/6 normal mice (P<0.01); the average wet weight of bilateral perigonadal adipose tissue in adult C57BL/6 normal mice (including male and female) was 1.24 g, the average wet weight of bilateral perigonadal adipose tissue in adult Glce mutant homozygous mice (including male and female) was 2.98 g, so there was a statistically significant difference (P<0.01) as compared with adult C57BL/6 normal mice.

The above results fully indicated that the wet weight of bilateral perigonadal adipose tissue in the adult Glce mutant homozygous mice was significantly increased as compared with the adult C57BL/6 normal mice.

### 5.2 Analysis of bilateral perirenal adipose tissue in Glce mutant homozygous mice

Glce mutant homozygous mice were dissected and bilateral perirenal adipose tissue was weighed on an electronic balance to study the difference in wet weight of bilateral perirenal adipose tissue between adult Glce mutant homozygous mice and adult C57BL/6 normal mice..

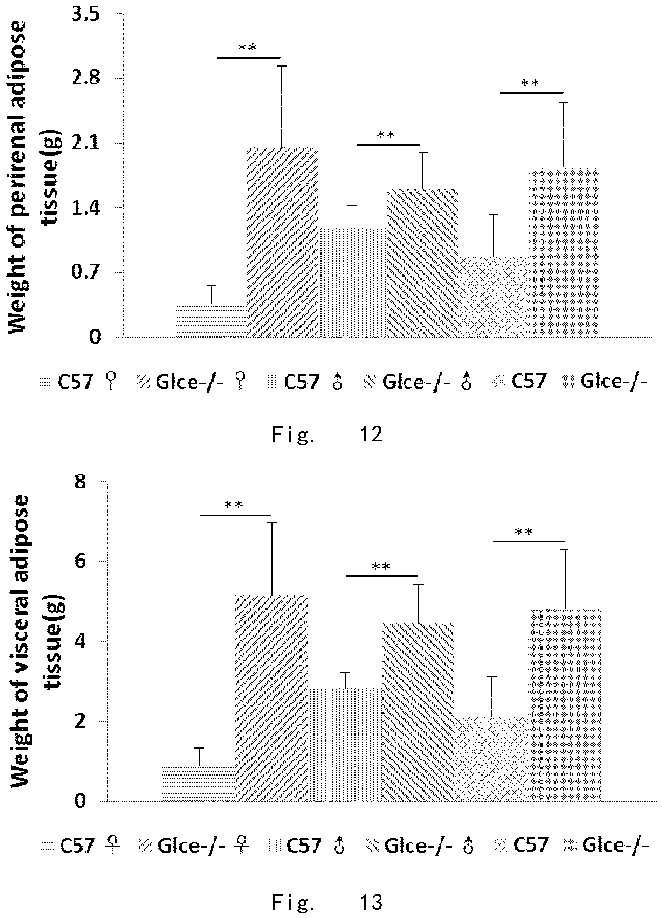

The wet weight of bilateral perirenal adipose tissue was significantly increased in adult Glce mutant homozygous mice as compared with adult C57BL/6 normal mice (Fig. 12).

The results showed that the average wet weight of bilateral perirenal adipose tissue in female adult C57BL/6 normal mice was 0.36 g, and the average wet weight of bilateral perirenal adipose tissue in female adult Glce mutant homozygous mice was 2.06 g, so there was a statistically significant difference as compared with female adult C57BL/6 normal mice (P<0.01). The average wet weight of bilateral renal adipose tissue in male adult C57BL/6 normal mice was 1.18 g. The average wet weight of bilateral perirenal adipose tissue in male adult Glce mutant homozygous mice was 1.61 g. There was a statistically significant difference as compared with male adult C57BL/6 normal mice (P<0.01). The average wet weight of bilateral perirenal adipose tissue in adult C57BL/6 normal mice (including male and female) was 0.87 g, and the average wet weight of bilateral perirenal adipose tissue in adult Glce mutant homozygous mice (including male and female) was 1.83 g, so there was a statistically significant difference (P<0.01) as compared with adult C57BL/6 normal mice.

The above results fully indicated that the wet weight of bilateral perirenal adipose tissue was significantly increased in adult Glce mutant homozygous mice as compared with adult C57BL/6 normal mice.

### 5.3 Analysis of visceral adipose tissue in Glce mutant homozygous mice

The Glce mutant homozygous mice were dissected and bilateral perigonadal adipose tissue and bilateral perirenal adipose tissue were weighed on an electronic balance. The total weight of visceral adipose tissue wet weight was the sum of wet weight of the bilateral perigonadal adipose tissue and the bilateral perirenal adipose tissue. The difference in wet weight of visceral adipose tissue between adult Glce mutant homozygous mice and adult C57BL/6 normal mice was studied.

The wet weight of visceral adipose tissue was significantly increased in adult Glce mutant homozygous mice as compared with adult C57BL/6 normal mice (Fig. 13).

The results showed that the average wet weight of visceral adipose tissue in female adult C57BL/6 normal mice was 0.90 g, and the average wet weight of visceral adipose tissue in female adult Glce mutant homozygous mice was 5.16 g. There was a statistically significant difference as compared with the female adult C57BL/6 normal mice (P<0.01). The average wet weight of visceral adipose tissue in male adult C57BL/6 normal mice was 2.84 g, and the average wet weight of visceral adipose tissue in male adult Glce mutant homozygous mice was 4.45 g. There was a statistically significant difference (P<0.01) as compared with male adult C57BL/6 normal mice. The adult C57BL/6 normal mice (including male and female) had an average wet weight of visceral adipose tissue of 2.11 g, and adult Glce mutant homozygote mice (including male and female) had an average wet weight of visceral adipose tissue of 4.81g. There was a statistically significantly difference as compared with the adult C57BL/6 normal mice (P<0.01).

The above results fully indicated that the wet weight of visceral adipose tissue in adult Glce mutant homozygous mice was significantly increased as compared with adult C57BL/6 normal mice.

### 5.4 Analysis of subcutaneous adipose tissue (such as bilateral inguinal adipose tissue) in Glce mutant homozygous mice

The Glce mutant homozygous mice were dissected and the bilateral inguinal adipose tissue was weighed on an electronic balance. The wet weight of subcutaneous adipose tissue was the sum of wet weight of the bilateral inguinal adipose tissue. The difference in wet weight of subcutaneous adipose tissue between adult Glce mutant homozygous mice and adult C57BL/6 normal mice was studied.

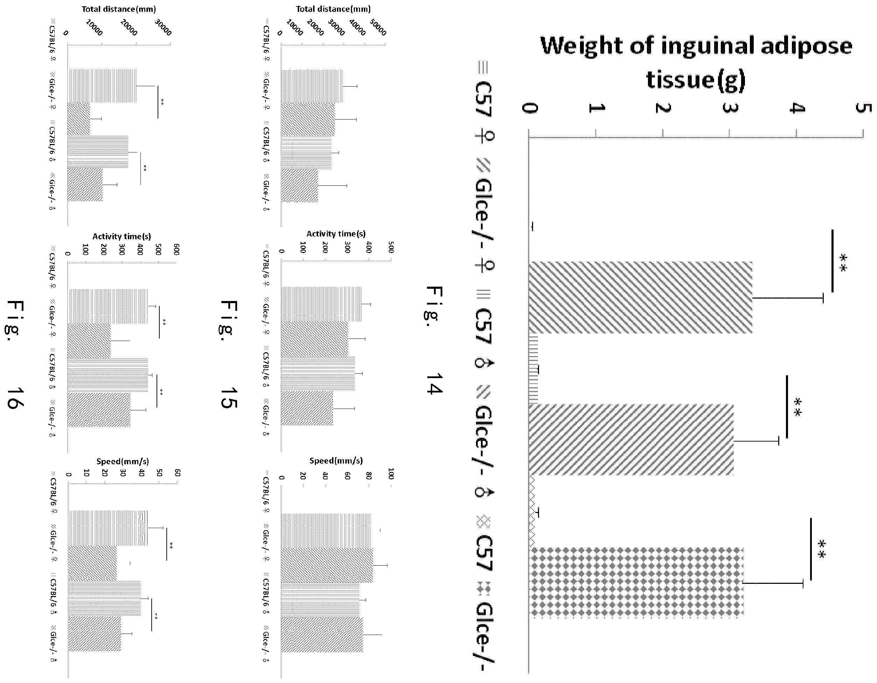

The wet weight of bilateral inguinal adipose tissue (representing subcutaneous adipose tissue) was significantly increased in adult Glce mutant homozygous mice as compared with adult C57BL/6 normal mice (Fig. 14).

The results showed that the average wet weight of bilateral inguinal adipose tissue in female adult C57BL/6 normal mice was 0.04 g, and the average wet weight of bilateral inguinal adipose tissue in female adult Glce mutant homozygous mice was 3.34 g, so there was a statistically significant difference as compared with the female adult C57BL/ 6 normal mice (P<0.01); the average wet weight of bilateral inguinal adipose tissue in male adult C57BL/6 normal mice was 0.13 g, and the average wet weight of bilateral inguinal adipose tissue in male adult Glce mutant homozygous mice was 3.07g, so there was a statistically significantly difference as compared with male adult C57BL/6 normal mice (P<0.01). The adult C57BL/6 normal mice (including male and female) had an average wet weight of bilateral inguinal adipose tissue of 0.10 g, and adult Glce mutant homozygous mice (including male and female) had an average wet weight of bilateral inguinal adipose tissue of 3.20 g, so there was a statistically significantly difference as compared with adult C57BL/6 normal mice (P<0.01).

The above results fully indicated that the wet weight of bilateral inguinal adipose tissue (representing subcutaneous adipose tissue) was significantly increased in adult Glce mutant homozygous mice as compared with adult C57BL/6 normal mice.

### Example 6 Depression behavioral analysis of Glce mutant homozygous mice

### 6.1 Independent activities

The Glce gene-mutated homozygous mice carrying the Cre recombinase were placed in a dark experimental chamber (110 mm X 110 mm X 330 mm), and the spontaneous activity of mice was tested for 5 min. The activity of mice was photographed by infrared imaging. The mouse trajectory and activity time were analyzed using a video tracking and analysis software purchased from Shanghai Jiliang Software Technology Co., Ltd.

Studies had shown that, there was no significant difference in the total distance, activity time, and activity speed of spontaneous activity in adolescent Glce mutant homozygous mice as compared with adolescent C57BL/6 normal mice (Figure 15).

The results showed that there was no significant difference in the natural habits of adolescent Glce mutant homozygous mice as compared with C57BL/6 normal mice.

### 6.2 open field experiment

The mice were placed in a bright open experiment box (500 mm X 500 mm X 590 mm) and the exploratory activity of the mice was tested for 5 min. The activity of the mice was photographed by infrared imaging. The mouse trajectory and activity time were analyzed using a video tracking and analysis software purchased from Shanghai Jiliang Software Technology Co., Ltd.

The results showed that, when compared with the adolescent C57BL/6 normal mice, the total distance, activity time and activity speed of the adolescent Glce mutant homozygous mice in the open field experiment were significantly reduced (Fig. 16). The results showed that the activity of adolescent Glce mutant homozygous mice was significantly reduced as compared with C57BL/6 normal mice.

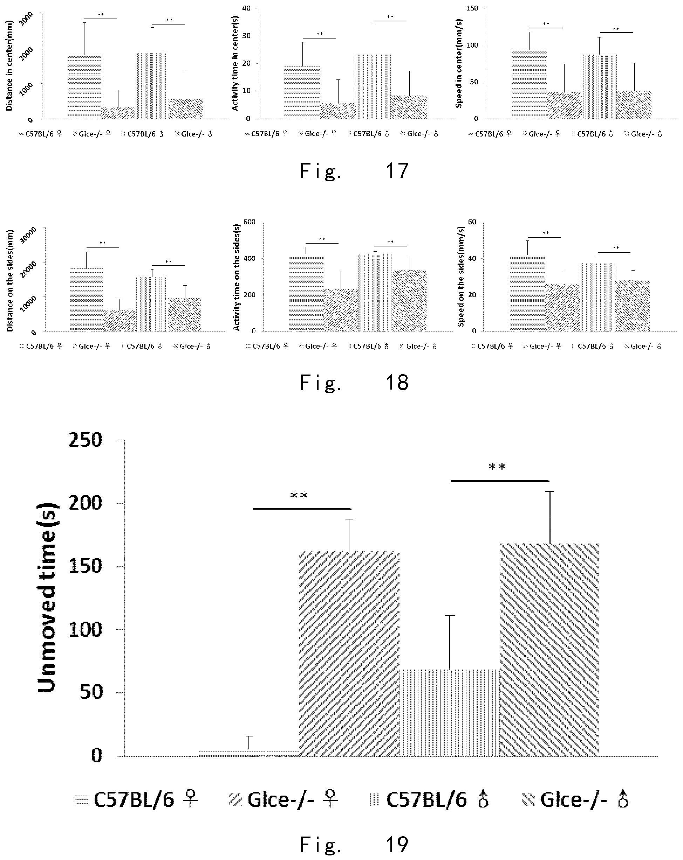

When compared with adolescent C57BL/6 normal mice, the activity distance, activity time and activity speed of adolescent Glce mutant homozygous mice in the central area of the open field were significantly reduced (Fig. 17), and the results showed that the activity to explore new environments of adolescent Glce mutant homozygous mice was significantly reduced as compared with C57BL/6 normal mice..

When compared with adolescent C57BL/6 normal mice, the activity distance, activity time and activity speed of adolescent Glce mutant homozygous mice in the surrounding area of the open field were significantly reduced (Fig. 18), and the results showed that the activity of adolescent Glce mutant homozygous mice was significantly reduced as compared with C57BL/6 normal mice.

Overall, as compared with adolescent C57BL/6 normal mice, the activity of adolescent Glce mutant homozygous mice was significantly reduced, and the activity of exploring new environment was significantly reduced, indicating that adolescent Glce mutant homozygous mice have a certain degree of depression.

### 6.3 Forced swimming

The mice were placed in a water tank (12 cm in diameter and 25 cm in height) and water temperature was 21-22 °C. The mice were forced to swim in water with low water temperature. The activity of mice was tested for 6 min, and the immobility time of the mice in the last 4 min was recorded. The activity of the mice was photographed by camera. The activity time of the mice was analyzed. Studies showed that the immobility time of adolescent Glce mutant homozygous mice in forced swimming experiments was significantly increased as compared with adolescent C57BL/6 normal mice (Figure 19).

The results showed that adolescent Glce mutant homozygous mice had a certain degree of depression as compared with C57BL/6 normal mice.

### Example 7 Stroke Behavior Analysis of Glce Mutant Homozygous Mice

Glce gene mutant homozygous mice carrying Cre recombinase were housed in a clean feeding environment. The mouse animal model developed a stroke-like symptom in the old age (about 1.5 years). The stroke symptoms and severity of the mouse model were evaluated by a series of stroke behavioral experiments. The experimental method was based on a series of systematic behavioral experiments on the severity of stroke symptoms published in Stroke in 2001 (Chen J, et al. Stroke. 2001), including exercise test, sensory test, balance ability test, body reflex test, abnormal activity test, etc., Specifically, behavior tests comprised such as flat test, tail-lifting test, visual tactile test, proprioceptive test, balance beam test, auricle reflex, eyelid reflex, startle reflex, convulsion, spasm, myodystony, etc., and the behavior of the mice was scored.

Only 10 of 40 mice in a Glce mutant homozygous mice group had a stroke in the old age, and the incidence of stroke was 25%; in the control group, 2 of 40 mice in C57BL/6 mouse group had a stroke in the old age, and the incidence of stroke was 5%. The results showed that Glce mutant homozygous mice were more likely to have a stroke than C57BL/6 mice.

### 7.1 Exercise test.

(1) Flat test: The mouse is placed on the ground to observe whether the mouse can walk normally, 0 points for normal walking; whether it can go straight, 1 point for not straight, otherwise 0 point; whether it circles around the injury side, 1 point for circling around the injury side, otherwise 0 point; whether it dumps to the injury side, 1 point for dumping to the injury side, otherwise 0 point.
(2) Tail-lifting test: The tail of the mouse is lifted to observe the movement of limbs of the mouse, including whether the forelimbs bent inward and the claws grasp, if the forelimbs of the mice bent inward and the paws grasp, the score is 1 point, otherwise the score is 0; whether the hind limbs bent inward and the claws grasp, if the hind limbs of the mouse bent inward and the paws grape and the score is 1 point, otherwise the score is 0 point; whether the head is raised within 30 seconds and the angle with the vertical axis is greater than 10 degrees, if the mouse is raised within 30 seconds and the angle with the vertical axis is greater than 10 degrees, the score is 1 point, otherwise the score is 0 point.

### 7.2 Feeling test.

(1) Visual tactile test: The mouse body is grasped, letting the forelimbs of the mouse move freely, making the mouse face the edge of table or cage, quickly bringing the mouse close to the edge of table or cage, and observing whether the forelimb of mouse can quickly stretch forward and open the claws in time to accurately grasp the edge of table or cage. If the forelimbs of mouse fail to quickly extend forward, or the claws fails to open in time to accurately grasp the edge of table or cage, the score is 1 point, otherwise the score is 0 point.
(2) Ontology test: The mouse body is grasped, letting the hind limbs of the mouse move freely, placing the forelimb of the mouse on the edge of table or cage, and hanging the hind limbs. A nipper is used to clip the hind leg muscles of the mouse so as to observe whether the hind limbs of the mouse can quickly retract. If the hind limbs of mice cannot retract quickly, the score is 1 point, otherwise the score is 0 point.

### 7.3 Balance ability test.

The mouse is placed at one end of the balance beam to observe the free movement of mouse on the balance beam. It mainly includes the following seven conditions: (1) whether it can maintain the body balance and walk freely on the balance beam. If the mouse can maintain the body balance and walk freely on the balance beam, then the score is 0 point.
(2) Whether it can grasp the edge of balance beam, and if the mouse grasps the edge of balance beam, the score is 1 point.
(3) Catching the balance beam, but there is one hind limb falling. If the mouse hugs the balance beam, but has a hind limb falling, then the score is 2 points.
(4) Catching the balance beam, but there are two hind limbs falling, or rotating on the balance beam, and the time on the balance beam is more than 60 seconds. If the mouse hugs the balance beam, but there are two hind limbs falling, or rotating on the balance beam, and the time on balance beam is greater than 60 seconds, then the score is 3 points.
(5) Mouse tries to balance on the balance beam but eventually falls, and the time on the balance beam is greater than 40 seconds. If the mouse tries to balance on the balance beam but eventually falls, and the time on the balance beam is greater than 40 seconds, the score is 4 points.
(6) Mouse tries to balance on the balance beam but eventually falls, and the time on the balance beam is greater than 20 seconds. If the mouse tries to balance on the balance beam but eventually falls and the time on the balance beam is greater than 20 seconds, then the score is 5 points.
(7) There is no attempt to balance on the balance beam or hug the balance beam and the time on the balance beam is less than 20 seconds. If the mouse does not attempt to balance on the balance beam or hug the balance beam, and the dropping time on the balance beam is less than 20 seconds, then the score is 6 points.

### 7.4 Body reflex test and abnormal activity test.

(1) auricle reflex: The cotton swab is used to stimulate the ear canal of the mouse to observe whether the mouse has a taro reaction. If yes, it indicates that the mouse has an auricle reflex; if no, it indicates that the mouse has no auricle reflex and the score is 1 point.
(2) corneal reflex: A cotton swab is used to stimulate the iris of the mouse to observe whether the mouse has a reaction to close the eyelid. If yes, it indicates that the mouse has a corneal reflex; if no, it indicates that the mouse has no corneal reflex, and the score is 1 point.
(3) startle reflex: A big noise, such as the water bottle falling, is made to observe whether the mouse has a shocked reaction. If yes, it indicates there is a startle reflex; if no, it indicates there is no startle reflection, and the score is 1 point.
(4) Observing whether the mouse has convulsions, spasms, myodystonia and other phenomena. If the mouse has convulsions, spasms, or myodystony, etc., the score is 1 point.

Calculating the total score of each item, the total score of 1-6 is defined as mild injuries, the total score of 7-12 is defined as moderate injuries, and the total score of 13-18 is defined as severe injuries.

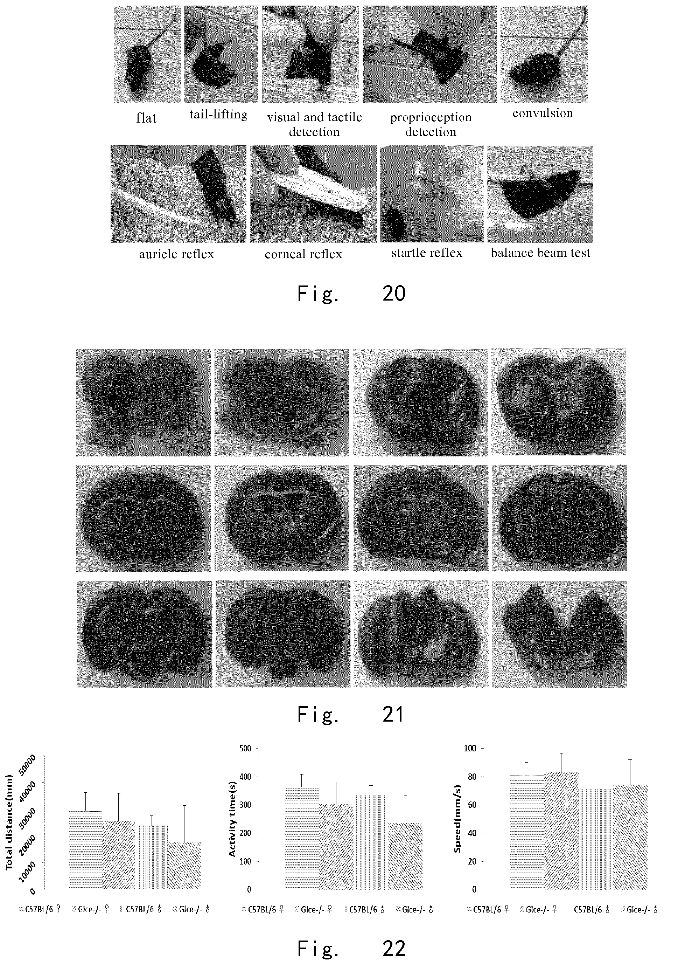

For example, as shown in Fig. 20, in the flat test, as the mouse could not go straight, it was scored 1 point, as it rotated around the injury side, it was scored 1 point and as it dumped to the side of the injury, it scored 1 point. In the tail-lasting test, as the forelimbs bended inward and the paws grasped, it was scored 1 point, as the hind limbs bended inward and the paws grasped, it was scored 1 point, and as the head was raised within 30 seconds and the angle to the vertical axis was greater than 10 degrees, it was scored 1 point. In the visual tactile test, as the forelimbs quickly extended forward and the claws opened in time to accurately grasp the edge of table or cage, it was scored 0 point. In the proprioceptive test, as hind limbs quickly retracted, it was scored 0 point. In the balance beam test, as the mouse attempted to balance on the balance beam but eventually felled, and the time on the balance beam was greater than 40 seconds, it was scored 4 points. The mouse had the auricle reflex, it was scored 0 point; the mouse had the corneal reflex, it was scored 0 point; and the mouse had the startle reflex, it was scored 0 point. The mouse was scored 1 point for convulsions. The total score of the mouse was: 1+1+1+1+1+1+0+0+4+0+0+0+1=11 points, which was evaluated as moderate stroke.

### Example 8 Analysis on brain tissue infarction in Glce mutant homozygous mice

The infarction of brain tissue of stroke mice was detected by TTC (2,3,5-triphenyltetrazolium chloride) staining. The operation steps were as follows: the brain was directly taken after anesthesia, and frozen in a -20 °C refrigerator for about 5-10 minutes before slicing. Slice operation: one slice was cut every 1 mm. The slices were placed in TTC at a conventional concentration of 2%. After covered with tin foil, it was put in a 37 °C incubator for 15-30 minutes, and the brain slices were turned over from time to time to make the brain slices evenly contact the staining solution. It was then fixed with 4% paraformaldehyde for 30 min. A photo was taken.

TTC is a fat-soluble, light-sensitive complex that can be used to stain for detecting ischemic infarction in mammalian tissues. It is a proton acceptor of the pyridine-nucleoside structural enzyme system in the respiratory chain, and reacts with dehydrogenase in normal tissues so as to appear red. When the activity of dehydrogenase in ischemic tissue decreases and can not react, there is no change so that it appears pale.

The results were shown in Fig. 21. The results showed that after TTC staining, the normal brain tissue of the stroke mice appeared red, and the infracted brain tissue appeared pale. Infarction was observed in the olfactory bulb, prefrontal cortex, corpus callosum, hippocampus, striatum, amygdala, hypothalamus, temporal lobe, cerebellum, pons, and medulla.

### Example 9 Anxiety Behavioral Analysis of Glce Mutant Homozygous Mice

Glce gene mutant homozygous mice carrying the Cre recombinase (hereinafter referred to as Glce mutant homozygous mice) were placed in a clean feeding environment. Anxiety-like symptoms in mouse animal models were evaluated in adolescent mice (> 3 months old) through a series of animal behavioral experiments including autonomic activity, open field experiment, and elevated cross maze.

### 9.1 Independent activity

The mice were placed in a dark experimental chamber (110 mm X 110 mm X 330 mm) and tested for autonomic activity of the mice for 5 min. The activity of mice was photographed by infrared imaging. The mouse trajectory and activity time were analyzed using a video tracking and analysis software purchased from Shanghai Jiliang Software Technology Co., Ltd.

Studies showed that there was no significant difference in the total distance, activity time, and activity speed of autonomic activity in adolescent Glce mutant homozygous mice as compared with adolescent C57BL/6 normal mice (Figure 22).

The results showed that there was no significant difference in the natural habits of adolescent Glce mutant homozygous mice as compared with C57BL/6 normal mice.

### 9.2 Open field experiment

The mice were placed in a bright open experiment box (500 mm X 500 mm X 590 mm) and the exploration activity of mice was tested for 5 min. The activity of mice was photographed by infrared imaging. The mouse trajectory and activity time were analyzed using a video tracking and analysis software purchased from Shanghai Jiliang Software Technology Co., Ltd.

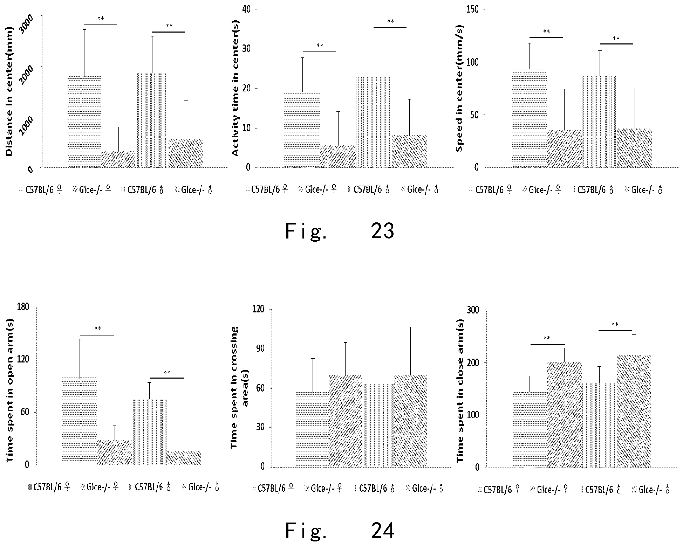

Studies showed that, as compared with adolescent C57BL/6 normal mice, the activity distance, activity time and activity speed of adolescent Glce mutant homozygous mice in the central area of the open field were significantly reduced (Figure 23).

The results indicated that, as compared with C57BL/6 normal mice, adolescent Glce mutant homozygous mice significantly reduced activity in new and different environments and produced more intense anxiety.

### 9.3 Elevated cross maze experiment

The mice were placed in an elevated cross maze with an open arm having a single arm length of 30 cm and a width of 6 cm, a closed arm having a single arm length of 30 cm and a width of 6 cm. The height of maze was 14.5 cm. The maze was placed at a height of about 50 cm from the ground. The activity of the mice in the elevated cross maze within 5 min was photographed by camera. Analysis on the mouse activity showed that, as compared with adolescent C57BL/6 normal mice, adolescent Glce mutant homozygous mice significantly reduced the time in open arms in the elevated cross maze test, and the time spent in the closed arm was significantly increased. There was no significant difference in the time spent in the central area (Figure 24).

The results indicated that the adolescent Glce mutant homozygous mice had a significantly increased anxiety as compared with C57BL/6 normal mice. Male adolescent Glce mutant homozygous mice were more anxious than female adolescent Glce mutant homozygous mice. Additionally, adolescent Glce mutant homozygous mice having a certain degree of anxiety also suffered from certain depression.

### Example 10 Verification of Screening Platform by using medicines for treatment of Obesity or Related Diseases (such as Hyperlipidemia)

In the present example, the model animal mice prepared in Example 1 were injected with the medicines, sibutramine or yam polysaccharide, which were used in the current clinical treatment of obesity or related diseases, and the severity of obesity in the model animal mice was evaluated.

The results showed that the medicines such as sibutramine or yam polysaccharide could significantly reduce the severity of obesity in the model animal mice and in particular, could significantly reduce the incidence of obesity symptom in the model animal mice, and/or adipose tissue wet weight (such as wet weight of bilateral perigonadal adipose tissue, wet weight of bilateral perirenal adipose tissue, wet weight of visceral adipose tissue, and/or wet weight of subcutaneous adipose tissue).

### Example 11 Screening medicine candidates for treating obesity or related diseases (such as hyperlipidemia) by using a medicine screening platform

In the present example, it was planned to inject a model animal mouse constructed in Example 1 with a therapeutic medicine for obesity or related diseases thereof and the severity of obesity in the model animal mice was evaluated.

By comparing the difference in obesity severity between model animal mice administered with the test medicine or placebo, the medicine candidate which can significantly reduce the incidence of obesity symptoms, and/or adipose tissue wet weight (such as wet weight of bilateral perigonadal adipose tissue, bilateral perirenal adipose tissue, visceral adipose tissue, and/or subcutaneous adipose tissue) will be a potential therapeutic medicine for treating obesity or related diseases.

For other obesity or related diseases, the above method can also be used to evaluate the severity of obesity in model animal mice, thereby screening out a medicine candidate.

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.

### References:

1. Kreuger J, Spillmann D, Li JP, Lindahl U. Interactions between heparan sulfate and proteins: the concept of specificity. The Journal of cell biology 2006;174: 323-7.
2. Li JP, Gong F, Hagner-McWhirter A, Forsberg E, Abrink M, Kisilevsky R, Zhang X, Lindahl U. Targeted disruption of a murine glucuronyl C5-epimerase gene results in heparan sulfate lacking L-iduronic acid and in neonatal lethality. The Journal of biological chemistry 2003;278: 28363-6.
3. Jia J, Maccarana M, Zhang X, Bespalov M, Lindahl U, Li JP. Lack of L-iduronic acid in heparan sulfate affects interaction with growth factors and cell signaling. The Journal of biological chemistry 2009;284: 15942-50.
4. Grigorieva E, Eshchenko T, Rykova VI, Chernakov A, Zabarovsky E, Sidorov SV. Decreased expression of human D-glucuronyl C5-epimerase in breast cancer. International Journal of cancer 2008;122: 1172-6.
5. Prudnikova TY, Mostovich LA, Domanitskaya NV, Pavlova TV, Kashuba VI, Zabarovsky ER, Grigorieva EV. Antiproliferative effect of D-glucuronyl C5-epimerase in human breast cancer cells. Cancer Cell international 2010;10: 27.
6. Grigorieva EV, Prudnikova TY, Domanitskaya NV, Mostovich LA, Pavlova TV, Kashuba VI, Zabarovsky ER. D-glucuronyl C5-epimerase suppresses small-cell lung cancer cell proliferation in vitro and tumor growth in vivo. British Journal of cancer 2011;105: 74-82.

## Claims

1. A method for preparing a non-human mammalian animal model of obesity or related diseases thereof, which comprises the steps of:
(a) providing a non-human mammalian cell, and inactivating a *Glce* gene in the cell, thereby obtaining a non-human mammalian cell with an inactivated *Glce* gene;
(b) using the cell with an inactivated *Glce* gene obtained in step (a) to obtain the animal model of obesity or related diseases thereof with the inactivated *Glce* gene.

2. The method of claim 1, wherein in step (a), it further comprises the following step:
(a1) deleting or interrupting one or more exons in exon 3 to exon 5 of *Glce* gene, and replacing with a selection marker by using the DNA homologous recombination techniques, thereby obtaining a non-human mammalian cell with the inactivated *Glce* gene.

3. The method of claim 1, wherein in step (b), it further comprises the following steps:
(b1) preparing and obtaining a chimeric non-human mammal by using the non-human mammalian cell with the inactivated *Glce* gene obtained in step (a);
(b2) mating the chimeric non-human mammal obtained in step (b1) with a normal wild-type non-human mammal, and screening progeny to obtain a heterozygous non-human mammal with an inactivated *Glce* gene;
(b3) mating the heterozygous non-human mammals obtained in step (b2) with each other to obtain a homozygous non-human mammal with the inactivated *Glce* gene, thereby obtaining the non-human mammalian animal model with the inactivated *Glce* gene.

4. The method of claim 3, wherein in step (b3), it further comprises a step of (b4): hybridizing the homozygous non-human mammal with the inactivated *Glce* gene with a neuron-specific knockout tool model which is the non-human mammal of the same species, thereby obtaining a non-human mammalian animal model with the inactivated neuron-specific *Glce* gene.

5. The method of claim 1, wherein, as compared with the wide type control animal, the non-human mammalian animal model with the inactivated Glce gene obtained in step (b) has one or more characteristics selected from the group consisting of:
(t1) an increase in degree of obesity;
(t2) an increase in incidence of obesity symptom;
(t3) an increase in wet weight of adipose tissue;
(t4) a decrease in level of open field activity;
(t5) a reduced desire to explore new environment;
(t6) an increase in depression-like behavior;
(t7) an increase in degree of depression;
(t8) an increase in anxiety-like behavior;
(t9) an increase in degree of anxiety;
(t10) an increased in fear-like behavior;
(t11) an increase in degree of fear;
(t12) an increase in cognitive disorder; and
(t13) an increase in incidence of stroke.

6. A use of a non-human mammalian animal model prepared by the method of claim 1 for studying obesity or related diseases thereof wherein it is used as an animal model.

7. A use of a non-human mammalian animal model prepared by the method of claim 1 for screening or identifying a substance (or a therapeutic agent) that relieves or treats obesity or related diseases thereof.

8. A method of screening or identifying a potential therapeutic agent for treating or relieving obesity or related diseases thereof, which comprises the steps of:
(a) in a test group, in the presence of a test compound, the test compound is administered to a non-human mammalian animal model prepared by the method of claim 1, and the severity Q1 of obesity or related diseases thereof in animal model of the test group is measured; and in the control group in which the test compound is not administered and the other conditions are the same, the severity Q2 of obesity or related diseases thereof in the animal model of the control group is measured;
(b) comparing the severity Q1 and severity Q2 measured in the previous step to determine whether the test compound is a potential therapeutic agent for treating or relieving obesity or related diseases thereof;
wherein, if the severity Q1 is significantly lower than the severity Q2, it indicates that the test compound is a potential therapeutic agent for treating or relieving obesity or related diseases thereof.

9. A use of a cell in which a Glucuronyl C5-epimerase (Glce) gene is inactivated or down-regulated for preparing a biological formulation for construction of a non-human mammalian animal model of obesity or related diseases.

10. A use of an inactivating agent of *Glce* gene or protein thereof for preparing a formulation for constructing a non-human mammalian animal model of obesity or related diseases.
